Europäisches Patentamt

European Patent Office

Office européen des brevets

(11) Publication number: **0 469 517 A2**

# EUROPEAN PATENT APPLICATION

(21) Application number: **91112712.4**

(22) Date of filing: **29.07.91**

(51) Int. Cl.5: **C12P 13/14**, C12N 1/20,
//(C12N1/20,C12R1:15,1:13)

The applicant has filed a statement in accordance with Rule 28 (4) EPC (issue of a sample only to an expert). Accession number(s) of the deposit(s): FERM BP-3004 and FERM BP-3005.

(30) Priority: **30.07.90 JP 210798/90**

(43) Date of publication of application:
**05.02.92 Bulletin 92/06**

(84) Designated Contracting States:
**DE ES FR GB IT**

(71) Applicant: **KYOWA HAKKO KOGYO CO., LTD.**
**6-1, Ohte-Machi Itchome**
**Chiyoda-ku Tokyo(JP)**

(72) Inventor: **Azuma, Tomoki**
**21-Clarendon Road**
**Belmont, MA 02178(US)**
Inventor: **Kuratsu, Yoshiyuki**
**2-2-302, Kyowa-cho**
**Hofu-shi, Yamaguchi-ken(JP)**

(74) Representative: **Vossius & Partner**
**Siebertstrasse 4 P.O. Box 86 07 67**
**W-8000 München 86(DE)**

(54) **Process for producing L-glutamic acid.**

(57) Disclosed is a process for producing L-glutamic acid by culturing a Coryneform glutamic acid-producing bacterium showing penicillin sensitivity in a medium until L-glutamic acid is accumulated in the medium; and recovering L-glutamic acid therefrom. L-Glutamic acid which is useful as drugs and food additives can be efficiently produced without performing operations of supplementing antibiotics, etc., and without inhibiting the L-glutamic acid productivity by the existence of an excess biotin contained in a medium.

EP 0 469 517 A2

The present invention relates to a process for producing L-glutamic acid by fermentation. L-Glutamic acid is used for various purposes over a wide range of the fields such as foods and drugs.

Where L-glutamic acid is produced by fermentation, L-glutamic acid productivity of L-glutamic acid-producing microorganisms which require biotin for growth extremely depends on biotin concentration in a medium. L-Glutamic acid can be produced in a specific limited concentration of biotin for growth. On the other hand, blackstrap molasses and starch hydrolyzate to be utilized as crude raw materials for inexpensive media contain an excess amount of biotin. A method for producing L-glutamic acid in such a medium containing an excess amount of biotin needs a operation such as supplementing an antibiotic, e.g. penicillin; or a surfactant during culturing, or elevating the culturing temperature. For producing L-glutamic acid by culturing a microorganism in a medium containing an excess amount of biotin without these operations, there are known a method of using a lysozyme-sensitive strain (JP-B-27798/87) and a method of using a strain having resistance to a compound having a vitamin P activity (JP-A-164792/81).

As the process for industrially producing L-glutamic acid by use of inexpensive crude raw materials, it has been desired to develop a process for producing L-glutamic acid efficiently, without performing operations such as supplementing penicillin, a surfactant, or elevating the culturing temperature.

According to the present invention, L-glutamic acid can be produced in a high yield by using a Coryneform glutamic acid-producing bacterium having sensitivity to penicillin.

In the present invention, any microorganism can be used so long as it belongs to so-called Coryneform glutamic acid-producing bacterium such as a microorganism belonging to the genus Corynebacterium or Brevibacterium, and shows penicillin sensitivity and so long as the production of L-glutamic acid by the microorganism is not inhibited by the existence of an excess biotin contained in a medium. In general, such a strain showing penicillin sensitivity is selected from mutants obtained by mutating parent strains belonging to the genus Corynebacterium or Brevibacterium and being capable of producing L-glutamic acid. The penicillin as used herein refers to penicillins including penicillin G, F, K, O, V and X and a salt thereof.

The penicillin-sensitive strain used in the present invention may be induced by a conventional mutation such as treatment with N-methyl-N'-nitro-N-nitrosoguanidine.

To select the mutant suitable for the present invention from mutation-induced strains, strains which grow poorly or cannot grow in a penicillin concentration in which its parent strain can grow, are selected. Herein penicillin-sensitivity means that the minimum growth inhibitory concentration of penicillin is smaller than that of the parent strain. That the production of L-glutamic acid is not inhibited by the existence of an excess biotin contained in a medium means that inhibition of L-glutamic acid production in the presence of an excess biotin in a medium is substantially ignorable. More specifically, L-glutamic acid can be produced without being affected by an excess biotin. In the Coryneform glutamic acid-producing bacterium, production of L-glutamic acid is generally inhibited when 10 $\mu$g/l of biotin or more is present in a medium. Accordingly, an excess biotin refers to the extent that 10 $\mu$g/l of biotin or more is present in a medium.

The parent strain for the mutants of the present invention includes, for example, Coryneform glutamic acid-producing bacterium such as Corynebacterium glutamicum ATCC 13032, Corynebacterium acetoacidophilum ATCC 13870, Corynebacterium lilium ATCC 15990, Brevibacterium flavum ATCC 14067, Brevibacterium lactofermentum ATCC 13869 and Brevibacterium divaricatum ATCC 14020. In addition, various mutants having an improved L-glutamic acid productivity which are induced from wild strains described above may also be used. In many cases, the yield may further increase by imparting a property conventionally known for increasing L-glutamic acid productivity, to the mutants of the present invention.

Hereafter a specific process for obtaining the mutant of the invention and the degree of penicillin sensitivity of the thus induced mutant are shown below.

Each of Corynebacterium glutamicum ATCC 13032 and Brevibacterium flavum ATCC 14067 was suspended in 10 ml of Tris-maleate buffer solution (pH 6.0) in a density of $10^9$ cells/ml in a conventional manner and 500 $\mu$g/ml of N-methyl-N'-nitro-N-nitrosoguanidine was added to the suspension. The mixture was allowed to stand at 30°C for 30 minutes. After collecting the cells, the cells were thoroughly washed with Tris-maleate buffer solution and smeared on medium A (1% peptone, 0.5% meat extract, 0.5% yeast extract, 0.25% NaCl, 2% agar, pH 7.2) followed by culture at 30°C for 48 hours. The thus obtained mutants were replicated in medium A and medium B obtained by supplementing 0.01 U/ml of penicillin G (potassium salt) to medium A. After culturing at 30°C for 48 hours, a mutant which grew poorly or did not grow in the penicillin-supplemented medium (medium B) but grew well in the medium supplemented with no penicillin (medium A) was harvested as the penicillin-sensitive strain. The parent strain showed good growth in either of the media. The mutant strain harvested in the present invention and its parent strain were cultured for 48 hours in medium A and medium B to compare the degree of growth of each strain. The results are shown in Table 1.

## Table 1

| Medium | Degree of Growth | | | |
|--------|------------|--------|------------|--------|
|        | ATCC 13032 | H-7684 | ATCC 14067 | H-7685 |
| A      | ++         | ++     | ++         | ++     |
| B      | ++         | -      | ++         | -      |

++ : well-growing

- : no growth

The penicillin-sensitive strains described above have been deposited with the Fermentation Research Institute, Agency of Industrial Science and Technology, Japan on July 12, 1990, respectively, under accession numbers Corynebacterium glutamicum H-7684 (FERM BP-3004) and Brevibacterium flavum H-7685 (FERM BP-3005).

For culturing the microorganism used in the present invention, media generally used for production of amino acid by fermentation may be used. That is either synthetic medium or natural medium containing carbon sources, nitrogen sources, inorganic salts and growth factors, which can be assimilated by the microorganism may be used. As the carbon sources, sugars such as glucose, fructose, sucrose, molasses, starch and starch hydrolyzates;

various organic acids such as acetic acid, fumaric acid and citric acid; alcohols such as ethanol, methanol and glycerol may be used. Blackstrap molasses is preferably used. As the nitrogen sources, ammonia; various inorganic salts such as ammonium chloride, ammonium sulfate, ammonium acetate and ammonium phosphate; organic acid ammonium salts such as ammonium fumarate; amines such as ethylamine; nitrogen-containing compounds such as urea; peptone, meat extract, yeast extract, corn steep liquor, casein hydrolyzate, soybean cakes or its hydrolyzates, various microbial cells as used in amino acid and nucleic acid fermentation, or its digested product may be used. As the inorganic materials, potassium dihydrogen phosphate, dipotassium hydrogen phosphate, magnesium phosphate, magnesium sulfate, sodium chloride, ferrous sulfate, manganese sulfate, copper sulfate and calcium carbonate may be used. As the growth factors, vitamins such as biotin, thiamine, nicotinic acid and $\beta$-alanine and amino acids such as glutamic acid may also be used.

Culturing is carried out under aerobic conditions, e.g., by shaking culture or agitation submerged culture. Culturing temperature is in a range of 20 to 40 $^{\circ}$C, preferably 25 to 38 $^{\circ}$C. The pH of the medium is in a range of 5 to 9, and is preferably maintained at around neutrality. The pH is adjusted with urea, calcium carbonate, an inorganic or organic acid, an alkali solution, ammonia or a pH buffer solution. Culturing is completed generally in 1 to 7 days, whereby L-glutamic acid is produced and accumulated in the culture broth.

After completion of the culturing, the precipitates such as cells are removed from the culture broth. By using ion exchange treatment concentration, salting out and isoelectric deposition in combination, L-glutamic acid can be recovered from the culture broth.

The present invention is illustrated by the following Examples.

Example 1

At first, 250 ml of a production medium (pH 7.2) consisting of 50 g/l blackstrap molasses (calculated as glucose), 10 g/l ammonium sulfate, 2 g/l urea, 0.5 g/l $KH_2PO_4$, 0.5 g/l $MgSO_4 \cdot 2H_2O$ and 30 g/l $CaCO_3$ was prepared in a 2 $\ell$-Erlenmeyer's flask. Corynebacterium glutamicum ATCC 13032 or H-7684 strain was cultured at 30 $^{\circ}$C for 24 hours in a seed medium (pH 7.2) consisting of 20 g/l glucose, 10 g/l peptone, 10 g/l yeast extract and 5 g/l NaCl. Then, 20 ml of the seed culture broth was inoculated into 250 ml of the production medium described above. In the culture broth of ATCC 13032 strain, accumulation of L-glutamic acid was not observed at all, whereas in the culture broth of H-7684 strain, 25.0 mg/ml of L-glutamic acid was produced and accumulated.

From the culture broth of H-7684 strain, 1 liter of the supernatant was obtained by centrifugation and passed through a column packed with strongly acidic cationic exchange resin [Dowex 50 x 8 (Na type), manufactured by Dow Chemical Co., Ltd.]. L-glutamic acid was eluted with ammonia, isolated, purified, concentrated and crystallized to give 20.0 g of L-glutamic acid crystals.

Example 2

In a manner similar to Example 1, Brevibacterium flavum ATCC 14067 or H-7685 strain was cultured. The results are shown in Table 2

## Table 2

| Strain | Amount of L-glutamic acid accumulated (g/l) |
|---|---|
| ATCC 14067 | trace |
| H-7685 | 17.0 |

## Claims

1. A process for producing L-glutamic acid which comprises culturing a Coryneform glutamic acid-producing bacterium showing penicillin sensitivity in a medium until L-glutamic acid is accumulated in the medium, and recovering L-glutamic acid therefrom.

2. The process according to claim 1, wherein the bacterium is a microorganism belonging to the genus Corynebacterium or Brevibacterium.

3. The process according to claim 2, wherein the microorganism belongs to the species Corynebacterium glutamicum or Brevibacterium flavum.

4. The process according to claim 3, wherein the microorganism is Corynebacterium glutamicum H-7684 (FERM BP-3004) or Brevibacterium flavum H-7685 (FERM BP-3005).

5. The process according to claim 1, wherein the culturing is carried out at 20 to 40°C for 1 to 7 days.

6. A biologically pure culture of the microorganism Corynebacterium glutamicum H-7684 (FERM BP-3004) or Brevibacterium flavum H-7685 (FERM BP-3005)